# EUROPÄISCHE PATENTSCHRIFT

(11) **EP 2 697 123 B1**
(45) Veröffentlichungstag und Bekanntmachung des Hinweises auf die Patenterteilung: **04.11.2015**
(21) Anmeldenummer: 12707534.9
(22) Anmeldetag: 01.03.2012
(51) Int. Cl.: B65B 31/02, B65B 9/067, A61J 3/07, B65B 1/48, G01N 23/10

(54) **VORRICHTUNG ZUR KONTROLLE VON PHARMAZEUTISCHEN PRODUKTEN, INSBESONDERE VON HARTGELATINEKAPSELN**
DEVICE FOR CHECKING PHARMACEUTICAL PRODUCTS, IN PARTICULAR HARD GELATIN CAPSULES
DISPOSITIF DE CONTRÔLE DE PRODUITS PHARMACEUTIQUES, EN PARTICULIER DE CAPSULES DE GÉLATINE DURE

(30) Priorität: 13.04.2011 DE 102011007277
(43) Veröffentlichungstag der Anmeldung: 19.02.2014
(73) Patentinhaber: Robert Bosch GmbH, 70442 Stuttgart (DE)
(72) Erfinder: SCHLIPF, Jens, 71691 Freiberg A. N. (DE); RUNFT, Werner, 71364 Winnenden (DE); MAGA, Iulian, 71638 Ludwigsburg (DE); VOGT, Martin, 73614 Schorndorf (DE)
(86) Internationale Anmeldenummer: PCT/EP2012/053556
(87) Internationale Veröffentlichungsnummer: WO 2012/139811

(56) Entgegenhaltungen:
- WO-A1-2006/027793
- WO-A1-2006/106012
- US-A- 3 493 746

## Beschreibung

### Stand der Technik

Die Erfindung betrifft eine Vorrichtung zur Kontrolle von pharmazeutischen Produkten, insbesondere von Hartgelatinekapseln, nach dem Oberbegriff des Anspruchs 1.

Aus der WO 2006/106012A1 ist eine Vorrichtung zur Kontrolle von Hartgelatinekapseln nach dem Oberbegriff des Anspruchs 1 bekannt. Bei der bekannten Vorrichtung ist es vorgesehen, dass die Hartgelatinekapseln in Formatteilen angeordnet sind, die an einem Förderrad austauschbar befestigt sind. Während der Fertigung der Hartgelatinekapseln gelangen die Formatteile nach dem Befüllen in den Bereich einer Kontrollstation, wo die Formatteile radial nach außen (in Bezug zu einer vertikalen Drehachse des Förderrads) bewegt werden, um von einer Röntgenstrahlprüfeinrichtung durchleuchtet zu werden. Die Röntgenstrahleinrichtung durchstrahlt die Hartgelatinekapseln in einer zur Richtung der Längsachse der Hartgelatinekapseln parallelen Richtung. Infolge der Ausbildung der Formatteile aus Metall und einer Stufenbohrung zur Aufnahme der Hartgelatinekapseln in einer Aufnahmebohrung des Formatteils wird als nachteilhaft angesehen, dass nicht der gesamte Querschnitt der Hartgelatinekapseln überwacht bzw. geprüft werden kann.

Weiterhin ist es aus der US 3,493,746 bekannt, zu überprüfende Kapseln oder ähnliches in einem für Röntgenstrahlung durchlässigen Behälter in Form eines Rohres anzuordnen, wobei die Längsachse des Rohrs senkrecht zur Strahlungsrichtung einer Röntgenstrahlquelle angeordnet ist. Weiterhin ist das Rohr um seine Längsachse drehbar angeordnet, um die Position der zu durchleuchtenden Objekte anpassen zu können.

Eine weitere Vorrichtung ist aus der nachveröffentlichten DE 10 2010 038 544 A1 der Anmelderin bekannt. Bei der bekannten Vorrichtung ist bei einer Ausführungsform ein in einer vertikalen Drehachse angeordnetes, schrittweise gedrehtes Förderrad vorgesehen, an dessen Umfang Aufnahmesegmente mit Aufnahmebohrrungen zur Aufnahme jeweils einer Hartgelatinekapsel austauschbar befestigt sind. Zum Überprüfen der Hartgelatinekapseln im Bereich einer Röntgenstrahlungsquelle werden die Hartgelatinekapseln aus den Aufnahmebohrungen der Aufnahmesegmente mittels Aufnahmestößel in den Bereich einer oberhalb der Aufnahmesegmente angeordneten Sensoreinrichtung überschoben. Zur Führung der Hartgelatinekapseln im Bereich der Sensoreinrichtung dient dabei ein schachtförmiges Förderelement. Bevor die Hartgelatinekapseln in die Aufnahmebohrungen der Aufnahmesegmente eingeschoben werden, sind diese in einem Massenspeicher, z.B. in Form eines Vorratsbehälters, angeordnet. Es sind somit zwei Überführvorgänge für die Hartgelatinekapseln erforderlich, bevor diese von der Röntgenstrahlungsquelle durchstrahlt werden: Zunächst erfolgt das Überschieben bzw. Einsortieren der Hartgelatinekapseln aus dem Massenspeicher in die Aufnahmebohrungen der Aufnahmesegmente, und anschließend findet das Ausschieben der Hartgelatinekapseln aus den Aufnahmebohrungen der Aufnahmesegmente in den Bereich der Sensoreinrichtung statt. Ein derartiges Handling der Hartgelatinekapseln ist relativ aufwändig und birgt die Gefahr von Beschädigungen an den Hartgelatinekapseln.

### Offenbarung der Erfindung

Ausgehend von dem dargestellten Stand der Technik liegt der Erfindung die Aufgabe zugrunde, ein vereinfachtes Handling der Produkte zu ermöglichen, die insbesondere einen produktschonenderen Transport bis in den Bereich der Sensoreinrichtung ermöglicht. Darüber hinaus soll die Leistung gegenüber dem Stand der Technik erhöht werden.

Diese Aufgabe wird bei einer Vorrichtung zur Kontrolle von pharmazeutischen Produkten, insbesondere von Hartgelatinekapseln, mit den Merkmalen des Anspruchs 1 gelöst. Durch die erfindungsgemäße Ausbildung der Vorrichtung ist es insbesondere nicht mehr erforderlich, die Produkte zum Durchstrahlen aus den Aufnahmen des Förderrads herauszunehmen, sondern sie werden vielmehr wäh rend ihres Transports in Stillstandphasen der Vorrichtung durchleuchtet bzw. durchstrahlt. Dadurch wird nicht nur der Handlingsaufwand der Vorrichtung gegenüber dem Stand der Technik reduziert, sondern es ergibt sich dadurch zusätzlich auch die Möglichkeit einer erhöhten Leistung, da nur noch ein einziger Fördervorgang, das Bestücken der Aufnahmen des Förderrads mit den Produkten, berücksichtigt werden muss. Um die Produkte innerhalb des Förderrads durchleuchten bzw. durchstrahlen zu können, ist es weiterhin erfindungsgemäß vorgesehen, dass das Formatteil (zur Aufnahme der Produkte) aus einem für die Strahlung der Strahlungsquelle durchlässigen Material besteht. Ein derartiges Material bildet beispielsweise Kunststoff aus, wobei Formatteile aus Kunststoff darüber hinaus auch noch relativ einfach und preiswert in der Herstellung sind.

Vorteilhafte Weiterbildungen der Vorrichtung zur Kontrolle von pharmazeutischen Produkten sind in den Unteransprüchen aufgeführt.

In der Vorrichtung gemäß der Erfindung, ist es vorgesehen, dass das Förderrad in einer vertikalen Drehachse gelagert ist, und dass in dem Förderrad senkrecht angeordnete Aufnahmebohrungen für die Produkte ausgebildet sind. Eine derartige Anordnung entspricht im Wesentlichen der in der DE 10 2010 038 544 A1 dargestellten Ausbildung des Förderrads mit dem Unterschied, dass bei der Erfindung die Produkte innerhalb des Förderrads durchleuchtet bzw. durchstrahlt werden.

Eine weitere Möglichkeit zur Leistungssteigerung sieht vor, dass in der Aufnahmebohrung mehrere Produkte stehend übereinander angeordnet sind.

Weitere Vorteile, Merkmale und Einzelheiten der Erfindung ergeben sich aus der nachfolgenden Beschreibung bevorzugter Ausführungsbeispiele sowie anhand der Zeichnung.

Diese zeigt in
- Fig. 1: eine erste, nicht von der Erfindung umfasste Vorrichtung zur Kontrolle von pharmazeutischen Produkten in einer vereinfachten perspektivischen Darstellung,
- Fig. 2: ein Detail der Fig. 1 im Bereich der Röntgenstrahlungsquelle in einem Teilquerschnitt,
- Fig. 3: das Detail gemäß der Fig. 2 in einer perspektivischen Ansicht,
- Fig. 4: eine gegenüber der ersten Vorrichtung modifizierte Vorrichtung im Bereich der Röntgenstrahlungsquelle in perspektivischer Ansicht,
- Fig. 5: eine weitere modifizierte Vorrichtung in perspektivischer Ansicht,
- Fig. 6: eine gegenüber der Fig. 1 im Bereich der Zufördereinrichtung für die Produkte in die Aufnahmen des Förderrads modifizierte Vorrichtung in perspektivischer Ansicht und
- Fig. 7: eine vereinfachte, teilweise geschnittene Darstellung einer erfindungsgemäßen Vorrichtung unter Verwendung eines in einer vertikalen Drehachse gedrehten Förderrads.

Gleiche Bauteile bzw. Bauteile mit gleicher Funktion sind in den Figuren mit den gleichen Bezugsziffern versehen.

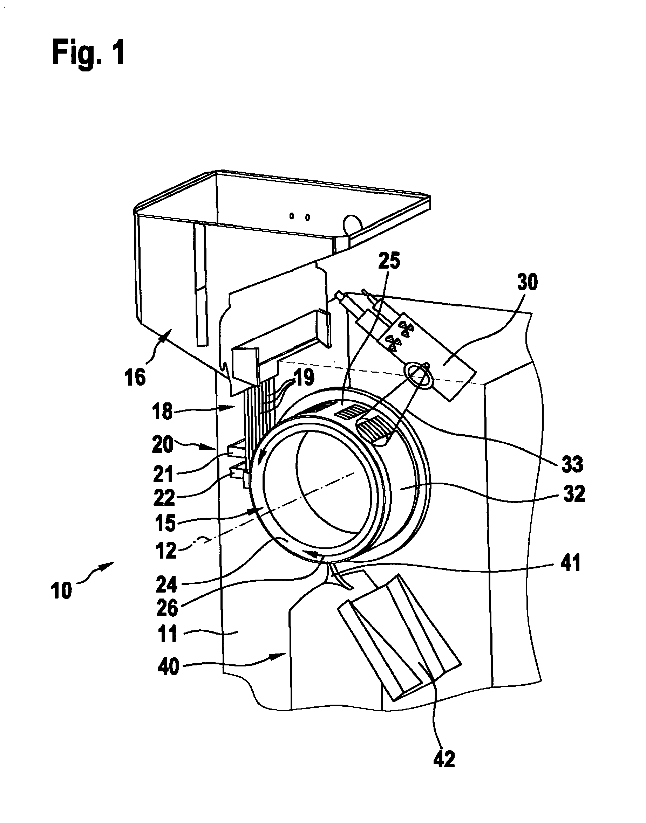

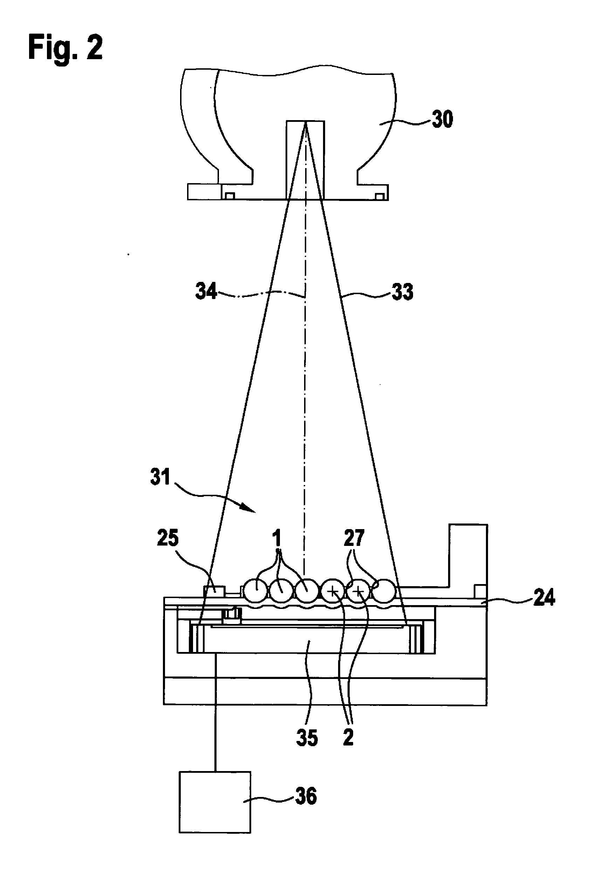

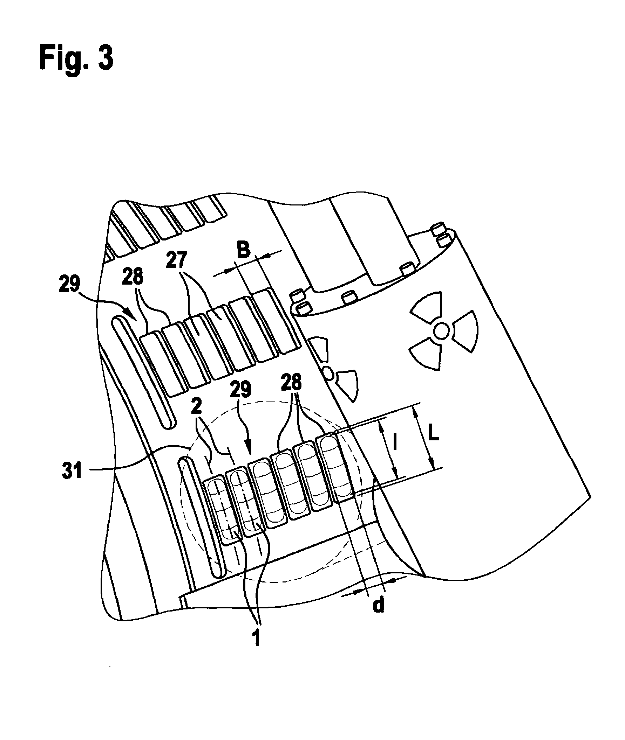

In den Fig. 1 bis 3 ist eine nicht von der Erfindung umfasste Vorrichtung 10 zur Kontrolle von pharmazeutischen Produkten, insbesondere zur Kontrolle von Hartgelatinekapseln 1 (siehe Fig. 2 und 3), dargestellt. Hierbei wird unter einer Kontrolle der Hartgelatinekapseln 1 bzw. der pharmazeutischen Produkte insbesondere eine Kontrolle auf ein korrektes Füllgewicht, sowie im Falle von Hartgelatinekapseln 1 eine Kontrolle auf das Vorhandensein von (nicht erwünschten) Fremdpartikeln verstanden.

Die Vorrichtung 10 umfasst ein Maschinengestell 11, aus dessen Vorderwand ein in einer horizontalen Drehachse 12 mittels eines nicht dargestellten Antriebs, insbesondere mittels eines Servomotors schrittweise gedrehtes Förderrad 15 herausragt. Seitlich oberhalb des Förderrads 15 ist ein Massenspeicher 16 für die Hartgelatinekapseln 1 in Form eines Trichters angeordnet. Zwischen dem Massenspeicher 16 und dem Förderrad 15 befindet sich ein dem Format der Hartgelatinekapseln 1 angepasstes Zuführmagazin 18, das mehrere, senkrecht zur Drehachse 12 ausgerichtete Aufnahmeschächte 19 aufweist. In den Aufnahmeschächten 19 sind die in dem Massenspeicher 16 ungeordneten Hartgelatinekapseln 1 in Form von jeweils als Reihe übereinanderstehende Hartgelatinekapseln 1 angeordnet, wobei das Zuführen der Hartgelatinekapseln 1 aus dem Massenspeicher 16 in die Aufnahmeschächte 19 des Zuführmagazins 18 auf an sich bekannte Art und Weise, zum Beispiel durch Vibration oder eine intermittierende Auf- und Abbewegung, erfolgt.

An dem dem Massenspeicher 16 abgewandten unteren Ende des Zuführmagazins 18 ist eine Sperreinrichtung 20 angeordnet, die beispielsweise zwei, in den Bereich der einzelnen Aufnahmeschächte 19 des Zuführmagazins 18 ein- und ausfahrbare Sperrstößel 21, 22 oder ähnliches aufweist. Mittels der Sperreinrichtung 20 lassen sich zumindest die jeweils untersten Hartgelatinekapseln 1 aus den Aufnahmeschächten 19 heraus in den Bereich des Förderrads 15 fördern bzw. ausschleusen.

Das Förderrad 15 weist, wie insbesondere aus einer Zusammenschau der Fig. 1 bis 3 erkennbar ist, einen ortsfest angeordneten, eine Auflagefläche für die Hartgelatinekapseln 1 ausbildenden Innenring 24, und einen gegenüber dem ortsfesten Innenring 24 drehbaren, den Innenring 24 radial umfassenden Außenring 25 auf. Der Außenring 25 wird mittels eines nicht dargestellten Antriebs entsprechend des Doppelpfeils 26 in und entgegen des Uhrzeigersinns gedreht. Wie insbesondere anhand der Fig. 2 und 3 erkennbar ist, weist der Innenring 24 an seiner äußeren Umfangsfläche entsprechend der Anzahl der Aufnahmeschächte 19 für jeden Aufnahmeschacht 19 eine in Umfangsrichtung umlaufende Führungsrille 27 auf, die der teilweise formflüssigen Aufnahme der Hartgelatinekapseln 1 dienen, und die beim Transport der Hartgelatinekapseln 1 eine Ausrichtung der Längsachsen 2 der Hartgelatinekapseln 1 mit den Führungsrillen 27 bewirken.

Radial beabstandet zum Innenring 24 ist der Außenring 25 angeordnet. Der Außenring 25 weist eine Vielzahl von Aufnahmen für die Hartgelatinekapseln 1 in Form von länglichen Durchbrüchen 28 auf, die fluchtend zu dem Führungsrillen 27 ausgerichtet sind. Hierbei sind jeweils eine der Anzahl der Führungsrillen 27 bzw. der Aufnahmeschächte 19 entsprechende Zahl an Durchbrüchen 28 zu einer Gruppe 29 von Durchbrüchen 28 zusammengefasst, wobei die zu einer Gruppe 29 gehörenden Durchbrüche 28 in Richtung der Drehachse 12 des Außenrings 25 neben- bzw. hintereinander angeordnet sind, wie dies insbesondere anhand der Fig. 3 erkennbar ist. Weiterhin sind über den Umfang des Außenrings 25 in jeweils bevorzugt gleichmäßigen Winkelabständen eine Vielzahl von Gruppen 29 von Durchbrüchen 28 angeordnet. Die Durchbrüche 28 am Außenring 25 dienen der Mitnahme der in den Führungsrillen 27 angeordneten Hartgelatinekapseln 1, wobei der radiale Abstand zwischen dem Innenring 24, genauer gesagt zwischen den Führungsrillen 27, und dem Außenring 25 geringer ist als die Dicke d der Hartgelatinekapseln 1. Bevorzugt ist die Länge L eines Durchbruchs 28 in Förderrichtung bzw. Drehrichtung des Außenrings 25 größer als die Länge I einer Hartgelatinekapsel 1 bzw. die Gesamtlänge der Hartgelatinekapseln 1, wenn gleichzeitig mehrere Hartgelatinekapseln 1 in einem Durchbruch 28 des Außenrings 25 angeordnet sind. Die Breite B eines Durchbruchs ist bevorzugt etwas breiter als die Dicke d bzw. der Durchmesser der Hartgelatinekapseln 1.

Der Außenring 25 ist zumindest in den Bereichen seines Umfangs, an denen die Hartgelatinekapseln 1 aus den Führungsrillen 27 aufgrund der erwähnten Geometrie der Durchbrüche 28 durch die Durchbrüche 28 herausfallen würden von Schutzabdeckungen 32 umgeben, so dass ein Herausfallen der Hartgelatinekapseln 1 in Folge von Schwerkraft durch die Durchbrüche 28 verhindert wird (Fig. 1).

In einer nicht dargestellten Ausführungsform der Vorrichtung 10 wäre es auch denkbar, dass die Durchbrüche 28 in Förderrichtung betrachtet eine derartige Breite B aufweisen, dass diese geringer ist als die Dicke d bzw. der Durchmesser der Hartgelatinekapseln 1, so dass alleine dadurch ein Herausfallen aus den Durchbrüchen 28 ebenfalls vermieden werden könnte.

Auf dem Förderweg der Hartgelatinekapseln 1 in dem Förderrad 15 (im Ausführungsbeispiel im Uhrzeigersinn) gelangen die Hartgelatinekapseln 1 in den kegelförmigen Strahlungsbereich 31 einer als Röntgenstrahlungsquelle 30 ausgebildeten Strahlungsquelle. Wie insbesondere anhand der Fig. 2 und 3 erkennbar ist, ist die Ausbildung bzw. Ausrichtung des Strahlungsbereichs 31 derart, dass während einer Stillstandsphase des Förderrads 15 sämtliche Hartgelatinekapseln 1 durchstrahlt werden, die sich in einer Gruppe 29 von Durchbrüchen 28 befinden, die im Bereich des Strahlungsbereichs 31 sind.

Gemäß der Erfindung ist die Ausrichtung der Röntgenstrahlungsquelle 30 bzw. dessen Strahlungskegels 33 derart, dass die Mittelachse 34 des Strahlungskegels 33 senkrecht zur Längsachse 2 der Hartgelatinekapseln 1 verläuft, d.h., dass die Hartgelatinekapseln 1 in einer Ebene senkrecht zur Längsachse 2 durchstrahlt werden.

Auf der dem Strahlungskegel 33 gegenüberliegenden Seite der Hartgelatinekapseln 1 ist wenigstens ein Sensorelement 35 angeordnet, das als bildaufnehmendes Sensorelement 35 ausgebildet ist, und das mit einer Auswerteeinrichtung 36 verbunden ist. Mittels des Sensorelements 35 wird ein Bild der durchstrahlten Hartgelatinekapseln 1 erzeugt, und dieses Bild wird der Auswerteeinrichtung 36 zugeführt. Mittels eines in der Auswerteeinrichtung 36 befindlichen Algorithmus erkennt die Auswerteeinrichtung 36, ob die durchstrahlten Hartgelatinekapseln 1 eine bestimmte erforderliche Eigenschaft, beispielsweise ein bestimmtes Füllgewicht, oder beispielsweise keine Fremdpartikel, aufweisen. Bezüglich der Einzelheiten der Auswertung derartiger von Sensorelementen 35 erzeugter Bilder wird diesbezüglich auf die DE 10 2010 038 544 A1 der Anmelderin verwiesen, die insofern Bestandteil dieser Anmeldung sein soll.

Das Sensorelement 35 ist in dem dargestellten Ausführungsbeispiel radial innerhalb des Innenrings 24 angeordnet, wozu der Innenring 24 zumindest im Bereich des Strahlungskegels 33 aus einem für die Strahlung der Röntgenstrahlungsquelle 30 durchlässigen Material, beispielsweise aus Kunststoff, besteht.

Auf dem weiteren Förderweg des Förderrads 15 schließt sich an die Röntgenstrahlungsquelle 30 eine Ausscheideeinrichtung 40 mit Ausscheideklappen 41 an. Im dargestellten Ausführungsbeispiel ist lediglich eine einzige Ausscheideklappe 41 dargestellt. Die Vorrichtung 10 weist jedoch eine entsprechend der Anzahl der Aufnahmeschächte 19 entsprechende Zahl von Ausscheideklappen 41 auf, die einzeln angesteuert werden können. Mittels der Ausscheideklappen 41 können in den jeweiligen Durchbrüchen 28 transportierte Hartgelatinekapseln 1 insbesondere durch eine entsprechende Stellung der Auswerfklappe 41 ausgeschieden, oder beispielsweise über eine Rutsche 42 einer nicht dargestellten

Kontrollwaage zugeführt werden. Bevorzugt ist die Ausscheideeinrichtung 40 in einem bezogen auf das Förderrad 15 unteren Bereich des Förderrads 15 angeordnet, so dass das Ausscheiden von Hartgelatinekapseln 1 aus den Durchbrüchen 28 beispielsweise alleine durch die Schwerkraft erfolgen kann.

Die soweit beschriebene Vorrichtung 10 arbeitet wie folgt: Während einer Stillstandsphase des Förderrads 15 werden aus den Aufnahmeschächten 19 jeweils die gewünschte Anzahl von Hartgelatinekapseln 1 in die in Ausrichtung mit den Ausgängen der Aufnahmeschächte 19 ausgerichteten Durchbrüche 28 eingebracht. Anschließend wird das Förderrad 15 schrittweise in den Bereich des Strahlungskegels 33 der Röntgenstrahlungsquelle 30 gefördert, wo mittels des Sensorelements 35 ein Bild der durchstrahlten Hartgelatinekapseln 1 aufgenommen wird.

Vorzugsweise wird das Bild erst ermittelt, wenn der Außenring 25 um einen gewissen Wegbetrag, beispielsweise 1 mm, entgegen der Förderrichtung Hartgelatinekapseln 1 zurückgedreht wurde. Dadurch wird ein vollständiges Freistellen der Hartgelatinekapseln 1 von den Durchbrüchen 28 des Außenrings 25 erzielt, so dass der Außenring 25 das Bild nicht beeinflusst.

Dieses Bild wird der Auswerteeinrichtung 36 zugeführt und ausgewertet. Sollte sich herausstellen, dass einzelne Hartgelatinekapseln 1 nicht die gewünschten Eigenschaften aufweisen ("Schlecht-Kapsel"), so werden diese Hartgelatinekapseln 1 im Bereich der Ausscheideeinrichtung 40 durch eine entsprechende Stellung der Ausscheideklappe 41 aus dem Förderrad 15 ausgeschieden. Demgegenüber werden Hartgelatinekapseln 1, die die gewünschten Eigenschaften ("Gut-Kapseln") aufweisen durch eine entsprechende Stellung der Auswurfklappe 41 nicht aus den Durchbrüchen 28 entfernt bzw. ausgeschieden, sondern einer weiteren Verarbeitung zugeführt.

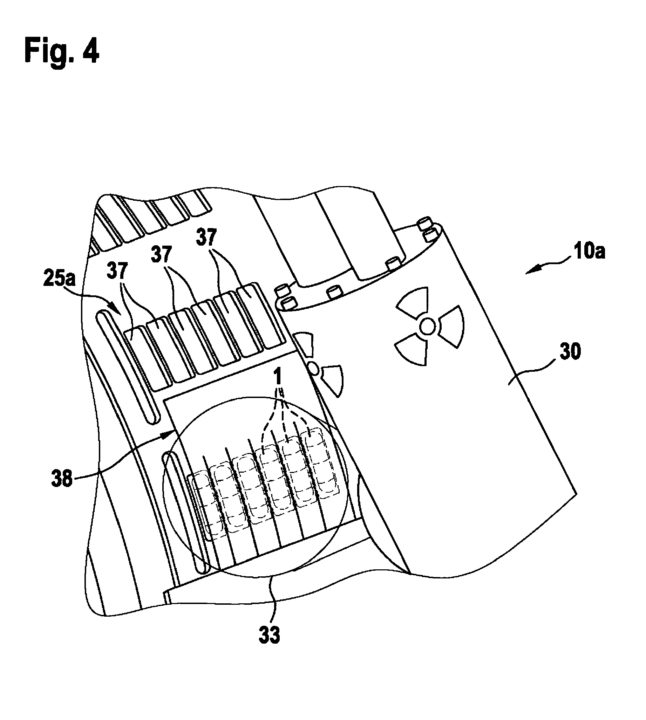

In der Fig. 4 ist eine gegenüber den Fig. 1 bis 3 modifizierte, ebenfalls nicht von der Erfindung umfasste Vorrichtung 10a dargestellt. Bei dieser Vorrichtung 10a gibt es im Gegensatz zur Vorrichtung 10 keinen ortsfest angeordneten Innenring 24, vielmehr weist der Außenring 25a in Analogie zu den Durchbrüchen 28 bei dem Außenring 25 Aufnahmen 37 für die Hartgelatinekapseln 1 auf, in denen die Hartgelatinekapseln 1 aufgenommen sind, ohne dass die beim Transport eine

Relativbewegung zu einem anderen Bauelement (wie beim Innenring 24 bei der Vorrichtung 10) ausführen. Weiterhin ist im Bereich der Röntgenstrahlungsquelle 30 bzw. des Strahlungskegels 33 ein für Röntgenstrahlung durchlässige Klemmplatte 38 angeordnet, die die Hartgelatinekapseln 1 gegen den Boden der Aufnahmen 37 drückt. Somit wird beim Rückwärtsdrehen des Außenrings 25a ein Festhalten der Hartgelatinekapseln 1 in den Aufnahmen 37 und somit ein Freistellen in Längsrichtung von den Aufnahmen 37 bewirkt.

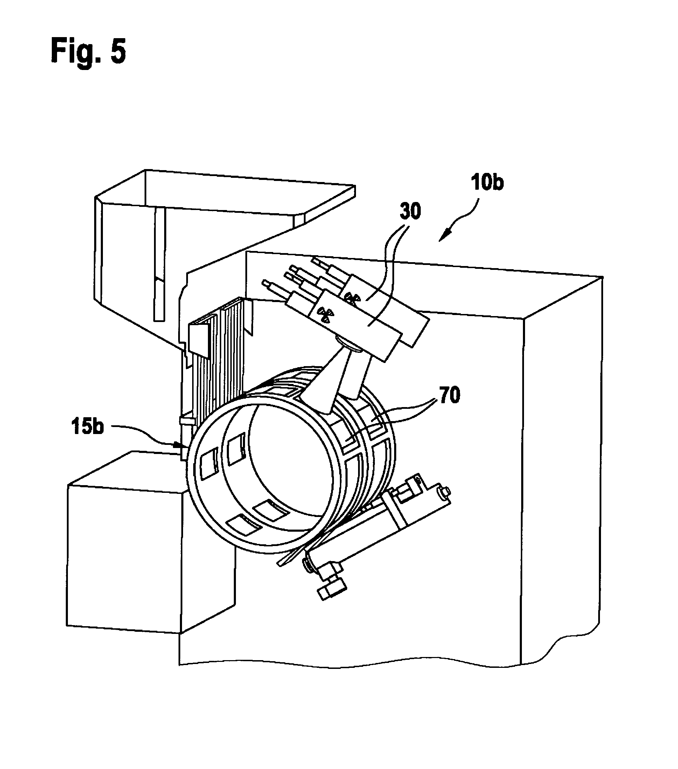

Bei der in der Fig. 5 dargestellten, nicht von der Erfindung umfassten Vorrichtung 10b weist das Förderrad 15b austauschbar angeordnete, als Formatteile für die Hartgelatinekapseln 1 ausgebildete Aufnahmeelemente 70 auf. In den Aufnahmeelementen 70 sind in Analogie zu den Aufnahmen 37 der Vorrichtung 10b Aufnahmen für die Hartgelatinekapseln 1 ausgebildet. Ferner weist die Vorrichtung 10b zwei parallel zueinander angeordnete Röntgenstrahlungsquellen 30 für in Drehrichtung parallel zueinander angeordnete Aufnahmeelemente 70 auf.

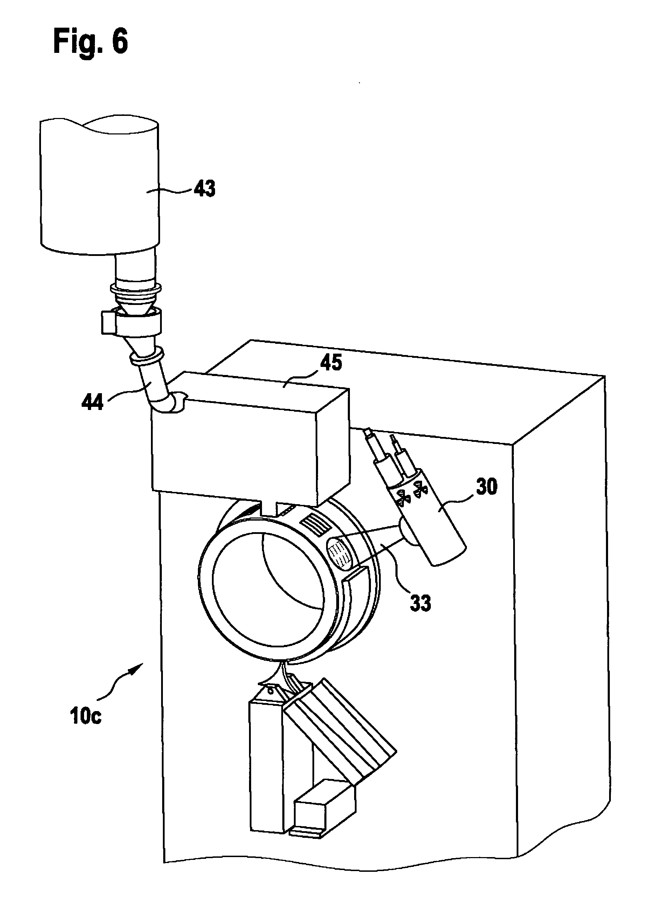

In der Fig. 6 ist eine weitere, gegenüber den Fig. 1 bis 4 modifizierte und nicht von der Erfindung umfasste Vorrichtung 10c dargestellt. Die Vorrichtung 10c weist einen Massenspeicher in Form eines Kapselvorratsbehälters 43 auf, der über ein Zuführrohr 44 mit einer formatunabhängigen Zufördereinrichtung 45 verbunden ist. Die beispielsweise pneumatisch betriebene Zufördereinrichtung 45 entnimmt aus dem Rohr 44, zum Beispiel mittels Vakuum bzw. Unterdruck, Hartgelatinekapseln 1 und fördert diese den Aufnahmen 37 bzw. Durchbrüchen 28 in dem Förderrad 15 zu.

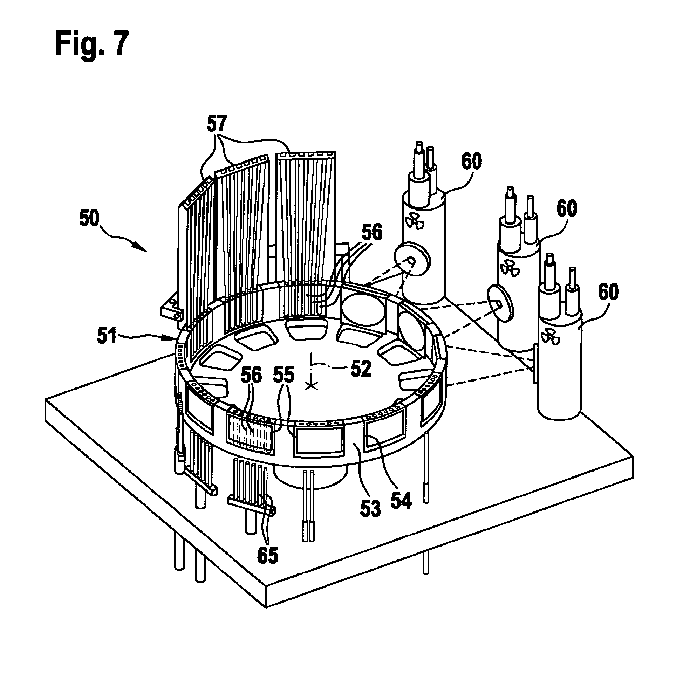

In der Fig. 7 ist eine erfindungsgemäße Vorrichtung 50 dargestellt. Die Vorrichtung 50 weist, im Gegensatz zu den Vorrichtungen 10, 10a, 10b ein Förderrad 51 auf, das in einer vertikal angeordneten Drehachse 52 schrittweise gedreht wird. An einer ringförmigen, vertikal ausgerichteten Außenwand 53 des Förderrads 51 sind in gleichmäßigen Winkelabständen Aufnahmen 54 zur austauschbaren Befestigung von Formatteilen 55 ausgebildet.

In den Formatteilen 55 sind jeweils mehrere, insbesondere ebenfalls vertikal ausgerichtete, als Aufnahmen für die Hartgelatinekapseln 1 wirkende Durchgangsbohrungen 56 ausgebildet, die jeweils eine Höhe bzw. Länge aufweisen, die es ermöglicht, jeweils mehrere Hartgelatinekapseln 1 übereinander als Reihe stehend in den Durchgangsbohrungen 56 aufzunehmen. Die Formatteile 55 bestehen aus einem Material, insbesondere aus Kunststoff, das für die Röntgenstrahlung durchlässig ist. Die Durchgangsbohrungen 56 der Formatteile 55 werden mittels schachtförmiger Zuführrinnen 57 aus einem nicht dargestellten Massenspeicher mit den Hartgelatinekapseln 1 befüllt, wobei im Bereich der Zuführrinnen 57 entsprechend der Vorrichtung 10 jeweils Sperreinrichtungen 20 angeordnet sind.

Im dargestellten Ausführungsbeispiel sind am Förderweg des Förderrads 51 außerhalb dessen Außenumfangs mehrere Röntgenstrahlungsquellen 60 angeordnet. Hierbei entspricht die Anzahl der Röntgenstrahlungsquellen 60 bevorzugt der Anzahl der Zuführrinnen 57, so dass das Förderrad 51 beispielsweise beim Vorhandensein von drei Zuführrinnen 57 jeweils schrittweise um einen Drehwinkelbereich weitergedreht wird, der der Teilung von drei Zuführrinnen 57 entspricht.

Um einzelne Hartgelatinekapseln 1, die als "schlecht" erkannt wurden, aus dem Förderrad 51 bzw. den Durchgangsbohrungen 56 der Formatteile 55 ausscheiden zu können, sind am weiteren Förderweg des Förderrads 51 nach den Röntgenstrahlungsquellen 60 entsprechend des Doppelpfeils 61 auf- und abbewegbare Ausscheidestößel 65 vorgesehen, die in die als Stufenbohrungen ausgebildeten Durchgangsbohrungen 56 der Formatteile 55 einfahren können, um damit die im Bereich der Durchgangsbohrung 56 befindlichen Hartgelatinekapseln 1 auszuscheiden.

Die soweit beschriebene erfindungsgemäße Vorrichtung 50 kann in vielfältiger Art und Weise abgewandelt bzw. modifiziert werden, ohne vom Erfindungsgedanken abzuweichen. Dieser besteht in der Verwendung eines Förderrades 51, in dem die Hartgelatinekapseln 1 bzw. pharmazeutischen Produkte während ihres Durchstrahlens durch die Röntgenstrahlungsquelle 60 gefördert bzw. angeordnet sind. So können insbesondere die Zuführeinrichtungen zum Zuführen der Hartgelatinekapseln 1 in die Förderräder 51 anders ausgebildet sein.

## Patentansprüche

1. Vorrichtung (50) zur Kontrolle von pharmazeutischen Produkten (1), insbesondere von Hartgelatinekapseln, mittels wenigstens einer vorzugsweise als Röntgenstrahlungsquelle ausgebildeten Strahlungsquelle (60), mit einer Fördereinrichtung, mittels der die Produkte (1) taktweise in einen Strahlungsbereich (31) der Strahlungsquelle (60) gefördert werden, wobei die Strahlung auf der der Strahlungsquelle (60) gegenüberliegenden Seite der Produkte (1) mittels wenigstens eines mit einer Auswerteeinrichtung (36) gekoppelten Sensorelements (35) erfasst wird, wobei
die Fördereinrichtung als ein schrittweise in einer vertikal angeordneten Achse (52) drehbares Förderrad (51) ausgebildet ist, wobei die Produkte (1) während ihrer Förderung in den Strahlungsbereich (31) in Aufnahmen des Förderrads (51) angeordnet sind, wobei die Aufnahmen als senkrecht angeordnete Aufnahmebohrungen (56) für die Produkte (1) ausgebildet sind, und wobei die Aufnahmebohrungen (56) in am Förderrad (51) austauschbar befestigten Formatteilen (55) angeordnet sind,
**dadurch gekennzeichnet,**
**dass** das Formatteil (55) aus einem für die Strahlung der Strahlungsquelle (60) durchlässigen Material, insbesondere aus Kunststoff, besteht, dass die Mittelachse (34) des Strahlungskegels (33) der Röntgenstrahlungsquellen (60) senkrecht zur Längsachse (2) der Produkte (1) verläuft, derart, dass die Produkte (1) in einer Ebene senkrecht zur deren Längsachse (2) durchstrahlt werden, und
**dass** die Produkte (1) innerhalb des Förderrads (51) durchstrahlt werden.

2. Vorrichtung nach Anspruch 1,
**dadurch gekennzeichnet,**
**dass** ein Formatteil (55) jeweils mehrere Aufnahmebohrungen (56) aufweist.

3. Vorrichtung nach Anspruch 1 oder 2,
**dadurch gekennzeichnet,**
**dass** am Förderweg des Förderrads (51) Ausschubstößel (65) zum Ausschieben von Produkten (1) aus der Aufnahmebohrung (56) vorgesehen sind.

4. Vorrichtung nach einem der Ansprüche 1 bis 3,
**dadurch gekennzeichnet,**
**dass** in der Aufnahmebohrung (56) mehrere Produkte (1) stehend übereinander angeordnet sind.

5. Vorrichtung nach einem der Ansprüche 1 bis 4,
**dadurch gekennzeichnet, dass** die Formatteile (55) an einer ringförmigen, vertikal ausgerichteten Außenwand (53) des Förderrads (51) in gleichmäßigen Winkelabständen angeordnet sind.

6. Vorrichtung nach einem der Ansprüche 1 bis 5,
**dadurch gekennzeichnet,**
**dass** am Förderweg des Förderrads (51) außerhalb dessen Außenumfangs mehrere Röntgenstrahlungsquellen (60) angeordnet sind.

## Claims

1. Device (50) for checking pharmaceutical products (1), in particular hard gelatine capsules, by means of at least one radiation source (60), preferably embodied as an x-ray radiation source, comprising a conveying apparatus, by means of which the products (1) are incrementally conveyed into a radiation region (31) of the radiation source (60), wherein the radiation is detected on the side of the products (1) lying opposite to the radiation source (60) by means of at least one sensor element (35) coupled to an evaluation apparatus (36), wherein
the conveying apparatus is embodied as a conveying wheel (51), rotatable in steps about a vertically disposed axis (52), wherein the products (1) are disposed in receptacles of the conveying wheel (51) during the conveyance thereof in the radiation region (31), wherein the receptacles are embodied as vertically disposed receptacle bores (56) for the products (1), and wherein the receptacle bores (56) are disposed in format parts (55) which are exchangeably fastened to the conveying wheel (51),
**characterized**
**in that** the format part (55) consists of a material, in particular a plastic, transmissive to the radiation of the radiation source (60), in that the central axis (34) of the radiation cone (33) of the x-ray radiation sources (60) extends perpendicular to the longitudinal axis (2) of the products (1) such that the products (1) are irradiated in a plane perpendicular to the longitudinal axis (2) thereof and
**in that** the products (1) are irradiated within the conveying wheel (51).

2. Device according to Claim 1,
**characterized**
**in that** a format part (55) respectively has a plurality of receptacle bores (56).

3. Device according to Claim 1 or 2,
**characterized**
**in that** discharge rams (65) for pushing products (1) out of the receptacle bore (56) are provided along the conveying path of the conveying wheel (51).

4. Device according to one of Claims 1 to 3,
**characterized**
**in that** a plurality of products (1) standing one above the other are disposed in the receptacle bore (56).

5. Device according to one of Claims 1 to 4,
**characterized**
**in that** the format parts (55) are disposed at a ring-shaped, vertically aligned outer wall (53) of the conveying wheel (51) at regular angular distances.

6. Device according to one of Claims 1 to 5,
**characterized**
**in that** a plurality of x-ray radiation sources (60) are arranged along the conveying path of the conveying wheel (51) outside of the external circumference thereof.

## Revendications

1. Dispositif (50) pour le contrôle de produits pharmaceutiques (1), en particulier de capsules de gélatine dure, au moyen d'au moins une source de rayonnement (60) réalisée de préférence sous forme de source de rayons X, comprenant un dispositif de transport au moyen duquel les produits (1) sont transportés de manière cadencée dans une région de rayonnement (31) de la source de rayonnement (60), le rayonnement sur le côté des produits (1) opposé à la source de rayonnement (60) étant détecté au moyen d'au moins un élément de capteur (35) accouplé à un dispositif d'analyse (36),
le dispositif de transport étant réalisé sous forme de roue de transport (51) pouvant tourner pas à pas dans un axe disposé verticalement (52), les produits (1) étant disposés pendant leur transport dans la région de rayonnement (31) dans des logements de la roue de transport (51), les logements étant réalisés sous forme d'alésages de réception (56) pour les produits (1) disposés verticalement, et les alésages de réception (56) étant disposés dans des parties de format (55) fixées de manière remplaçable sur la roue de transport (51),
**caractérisé en ce que**
la partie de format (55) se compose d'un matériau perméable au rayonnement de la source de rayonnement (60), en particulier de plastique, **en ce que** l'axe médian (34) du cône de rayonnement (33) des sources de rayons X (60) s'étend perpendiculairement à l'axe longitudinal (2) des produits (1), de telle sorte que les produits (1) soient traversés par le rayonnement dans un plan perpendiculaire à leur axe longitudinal (2), et
**en ce que** les produits (1) sont traversés par le rayonnement à l'intérieur de la roue de transport (51).

2. Dispositif selon la revendication 1,
**caractérisé en ce**
**qu'**une partie de format (55) présente à chaque fois plusieurs alésages de réception (56).

3. Dispositif selon la revendication 1 ou 2,
**caractérisé en ce que**
des poussoirs d'éjection (65) pour éjecter des produits (1) hors de l'alésage de réception (56) sont prévus sur la voie de transport de la roue de transport (51).

4. Dispositif selon l'une quelconque des revendications 1 à 3,
**caractérisé en ce que**
plusieurs produits (1) sont disposés debout les uns au-dessus des autres dans l'alésage de réception (56).

5. Dispositif selon l'une quelconque des revendications 1 à 4,
**caractérisé en ce que**
les parties de format (55) sont disposées à intervalles angulaires réguliers au niveau d'une paroi extérieure (53) orientée verticalement, de forme annulaire, de la roue de transport (51).

6. Dispositif selon l'une quelconque des revendications 1 à 5,
**caractérisé en ce que**
plusieurs sources de rayons X (60) sont disposées sur la voie de transport de la roue de transport (51) à l'extérieur de sa périphérie extérieure.
